# EUROPÄISCHE PATENTSCHRIFT

(11) **EP 0 796 607 B1**
(45) Veröffentlichungstag und Bekanntmachung des Hinweises auf die Patenterteilung: **12.02.2003**
(21) Anmeldenummer: 97250080.5
(22) Anmeldetag: 17.03.1997
(51) Int. Cl.: A61K 6/00, C08G 61/00

(54) **Funktionalisiertes und polymerisierbares Polymer**
Functionalized and polymerisable polymer
Polymère polymérisable et fonctionalisé

(30) Priorität: 20.03.1996 DE 19613017; 12.04.1996 DE 19616183
(43) Veröffentlichungstag der Anmeldung: 24.09.1997
(73) Patentinhaber: Ivoclar Vivadent AG, 9494 Schaan (LI)
(72) Erfinder: Rheinberger, Volker, Dr., 9490 Vaduz (LI); Moszner, Norbert, Prof. Dr., 9492 Eschen (LI); Stelzer, Franz, Prof. Dr., 8020 Graz (AT); Schitter, Regina, Dipl. Ing., 8850 Murau (AT); Zeuner, Frank, Dr., 9490 Vaduz (LI)
(74) Vertreter: UEXKÜLL & STOLBERG

(56) Entgegenhaltungen:
- EP-A- 0 219 058
- US-A- 4 054 233
- BELL B ET AL: "A ONE-POT SYNTHESIS OF COMB POLYMERS AND HYDROGELS BY THE RING-OPENING METATHESIS POLYMERIZATION REACTION" MACROMOLECULAR: RAPID COMMUNICATIONS, Bd. 15, Nr. 6, 1.Juni 1994, Seiten 543-550, XP000464089
- WECK M ET AL: "SYNTHESIS OF ABA TRIBLOCK COPOLYMERS OF NORBORNENES AND 7- OXANORBORNENES VIA LIVING RING-OPENING METATHESIS POLYMERIZATION USING WELL-DEFINED, BIMETALLIC RUTHENIUM CATALYSTS" MACROMOLECULES, Bd. 29, Nr. 5, 26.Februar 1996, Seiten 1789-1793, XP000555556
- KAPELLEN K K ET AL: "SYNTHESIS AND CHARACTERIZATION OF AMPHIPHILIC COMB-POLYMERS VIA RING-OPENING METATHESIS POLYMERIZATION OF EXI,EXO-5,6-BIS(ALKOXYMETHY L)-7-OXABICYCLO 2.2.1HEPT-2-ENES" POLYMER BULLETIN, Bd. 32, Nr. 1, 1.Januar 1994, Seiten 3-10, XP000420832

## Beschreibung

Die Erfindung betrifft ein funktionalisiertes und polymerisierbares Polymer, ein Verfahren zu dessen Herstellung, dessen Verwendung sowie Zusammensetzungen mit Gehalt an dem Polymer.

Durch Carboxylgruppen funktionalisierte Polymere, wie Poly(acrylsäure) und Poly(methacrylsäure) sowie Homo- und Copolymerisate auf der Basis von Maleinsäure oder Fumarsäure, finden in der Technik breite Anwendung. Sie werden unter anderem als Flockungs- oder Verdickungsmittel, als Komponente von Beschichtungen oder Klebstoffen sowie als Leder- oder Textilhilfsmittel eingesetzt (vgl. Ullmann's Encyclopedia of Industrial Chemistry, 5. Ed., Vol. A21, VCH Publisher, Weinheim 1992, S. 143 ff. und Encyclopedia of Polymer Science and Engineering, Vol. 9, Wiley & Sons, New York 1987, S. 225 ff.). Polyacrylsäure wird im Dentalbereich auch als Bestandteil von sogenannten Carboxylatzementen oder von Alumosilikat-Polyacrylsäure-Zementen verwendet (vgl. K. Körber, K. Ludwig, Zahnärztliche Werkstoffkunde und Technologie, Thieme-Verlag, Stuttgart-New York 1982, S. 57 ff.).

Weiter haben in den letzten Jahren die sogenannten Glasionomerzemente großes praktisches Interesse gefunden. Es handelt sich bei ihnen um Zemente, die aus Mischungen eines Ca-Al-F-Silikatglaspulvers mit einer wäßrigen Lösung, z.B. eines Acrylsäure-Maleinsäure-Copolymeren, hergestellt werden. Sie werden als Befestigungszemente, Füllungsmaterialien, Unterfüllungsmaterialien, Adhäsive oder Fissurenversiegler im Dentalbereich angewendet (vgl. A. D. Wilson, J. D. McLean, Glasionomerzement, Quintessens-Verlag, Berlin 1988, S. 21 ff.). Im Falle der sogenannten lichthärtenden Glasionomerzemente werden üblichen Glasionomerzementen noch polymerisierbare Vernetzermonomere und Initiator zugegeben, was zu einer Beschleunigung der Materialaushärtung und Verbesserung der mechanischen Eigenschaften führt. Dabei kann durch den Einsatz von Polycarbonsäuren, die seitenständige polymerisationsfähige Gruppen tragen, eine weitere Verbesserung der Materialeigenschaften erreicht werden. Solche Polycarbonsäuren können z.B. durch polymeranaloge Umsetzung von Polyacrylsäure mit Allylisocyanat oder 2-Isocyanatoethylmethacrylat (vgl. EP-B-323 120 und S. B. Mitra, Amer. Chem. Soc., Polym. Div., Polym. Prep. **32**, (1991) S. 517) oder z.B. durch Reaktion von Oligomaleinsäureanhydrid mit 2-Hydroxyethylmethacrylat (vgl. EP-B-219 058) hergestellt werden. Entsprechende Polymere können auch durch polymeranaloge Umsetzung von Polyacrylsäure mit Glycidylmethacrylat (vgl. US-A-3 872 047) erhalten werden. Bei allen diesen Reaktionen müssen jedoch die bekannten Nachteile polymeranaloger Umsetzungen, wie behinderte Zugänglichkeit der funktionellen Gruppen, Nichtabtrennbarkeit von Nebenprodukten oder das Ablaufen von Ringschluß- oder Vernetzungsreaktionen, in Kauf genommen werden (vgl. M. Fedtke, Reaktionen an Polymeren, Verlag für Grundstoffindustrie, Leipzig 1985, S. 17 ff.).

Weiter ist es bekannt, daß mono- oder bicyclische Alkene, wie z.B. Cyclopenten oder Norbornen, mit Katalysatoren der Olefinmetathese, z.B. MoO₃/Al₂O₃ oder WCl₆/(C₂H₅)₂Al, einer Ringöffnungspolymerisation unterworfen werden können. Dieser Reaktionstyp wird auch als Metathesepolymerisation bezeichnet (vgl. Encyclopedia of Polymer Science and Engineering, Vol. 9, J. Wiley & Sons, New York, 1987, S. 634 ff. und K. J. Ivin, Olefin Metathesis, Academic Press, London 1983). Auch bei polaren Verbindungen, wie z.B. 7-Oxa-bicyclo[2.2.1]hept-5-en-Derivaten, die in 2- oder 3-Stellung polare Substituenten, wie Alkoxy, Hydroxyalkyl, Alkoxycarbonyl, Carboxyl oder Carbonsäureanhydrid, aufweisen, ist in wäßrig-alkoholischem Reaktionsmedium mit Ruthenium(III)-chlorid als Katalysator eine ringöffnende Metathesepolymerisation (RÖMP) möglich (vgl. B. M. Novak, R. H. Grubbs, J. Amer. Chem. Soc. **110**, (1988) S. 960, 7542, W. J. Feast, D. B. Mallison, Polymer **32** (1991) S. 558 und A. Y. Lu et al. Makromol. Chem. Phys. **195** (1994) S. 1273). Weiter ist auch die RÖMP von Cyclooct-5-enylmethacrylat bekannt, die zu radikalisch vernetzbaren Polymeren führt (vgl. B. R. Maughon, R. H. Grubbs, Amer. Chem. Soc., Polym. Div., Polym. Prep. **36**, (1995) S. 471).

Aus B. Bell, J.G Hamilton, E.E. Law, J.J. Rooney, Macromol. Rapid Comm. 15, (1994) S. 543 - 550, sind Polymere die durch ring öffnende Metathesepolymerisation (RÖMP) aus verschiedenen Norbornen- oder Oxanorbornen Derivaten bekannt.

Außerdem sind auch bicyclische Methacrylate bekannt. So offenbart US-A-4 054 233 die Synthese und Polymerisation von Bicyclo[2.2.1]hept-5-en-2-ylmethylmethacrylat im Zusammenhang mit peroxidisch venetzbaren Schichten. Gemäß SU-A-1 776 673 und Chem. Abst. **199**, 272563, finden Bicyclo[2.2.1.]hept-5-en-2-ylmethylmethacrylat oder Borneolmethacrylat bei der Herstellung von PVC mit verbesserter Wärmestabilität Verwendung. CA-A-1 013 095 offenbart adhäsive Polymere auf der Basis von Umsetzungsprodukten von Bicyclo[2.2.1]hept-5-en-2,3-dicarbonsäureanhydridmit Hydroxyalkyl(meth)acrylaten, wie z.B. 2-Hydroxyethylmethacrylat. Aus T. M. Pyriadi, I. U. Altmamimi, Macromol. Rep. **A31**, (1994) S. 191, sind radikalisch vernetzbare Polyimide bekannt, welche über 7-Oxa-5,6-dicarboxyimid-N-yl-bicyclo[2.2.1]hept-2-enacrylat zugänglich sind. Schließlich sind auch Produkte der Umsetzung von Dicyclopentadien mit (Meth)acrylsäure bekannt (vgl. S. Teshigahara, Y. Kano, Toso Kenkyu Hokoko, **35** (1991) S. 47 und Chem. Abstr. **116**, 84740).

Der Erfindung liegt nunmehr die Aufgabe zugrunde, ein funktionalisiertes und polymerisierbares Polymer zur Verfügung zu stellen, das auf einfache Weise hergestellt und bei Raumtemperatur radikalisch polymerisiert werden kann, eine hohe Haftung auf verschiedenen Substraten zeigt, mit reaktiven Füllstoffen Zemente bildet und daher insbesondere als Komponente von Zementen, Beschichtungsmaterialien, Adhäsiven oder Kompositen und vorzugsweise von Dentalmaterialien eingesetzt werden kann. Diese Aufgabe wird durch das funktionalisierte und polymerisierbare Polymer nach den Ansprüchen 1 und 2 gelöst.

Gegenstand der vorliegenden Erfindung sind ebenfalls ein Verfahren zur Herstellung des Polymers nach Anspruch 3, dessen Verwendung nach den Ansprüchen 4 und 5 sowie Zusammensetzungen mit Gehalt an dem Polymer nach den Ansprüchen 6 bis 9.

Das erfindungsgemäße funktionalisierte und polymerisierbare Polymer zeichnet sich dadurch aus, daß es die folgenden wiederkehrenden Einheiten (IA), (IB) und (IC) aufweist: 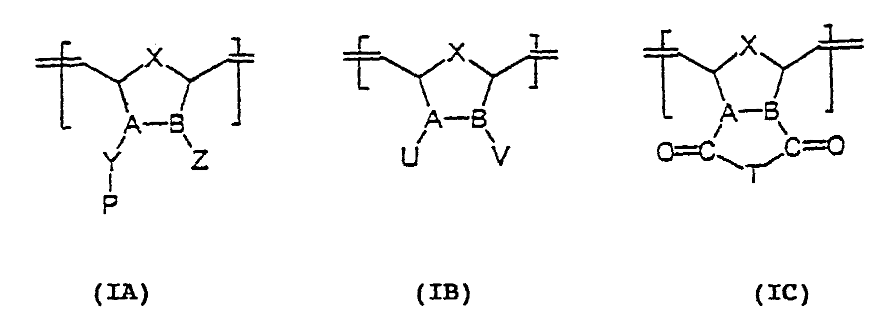 wobei X, A-B, Y, P, Z, U, V, T, R¹, R², R³, R⁴ und R⁵ unabhängig voneinander die folgenden Bedeutungen haben:
- X =: CH₂ oder O;
- A-B =: C-C oder C=C;
- Y =: CH₂O, CO-O oder COO-R¹-O,
wobei R¹ = substituiertes oder unsubstituiertes C₁- bis C₅-Alkylen oder -Oxyalkylen;
- P =: eine polymerisierbare Gruppe, nämlich CH₂=CH-CO-, CH₂=C(CH₃)-CO-, CH₂=CH-CH₂- oder CH₂=CH-C₆H₅-CH₂-;
- Z =: H, COOH, substituiertes oder unsubstituiertes C₁- bis C₁₂-Alkyl oder COOR⁴,
wobei R⁴ = substituiertes oder unsubstituiertes C₁- bis C₁₂-Alkyl oder C₆- bis C₁₄-Aryl,
- U =: COOH oder COOR⁵,
wobei R⁵ = substituiertes oder unsubstituiertes C₁- bis C₁₂-Alkyl oder C₆- bis C₁₄-Aryl;
- V =: H, COOH, CH₂-OH, OR² oder CO-OR²,
wobei R² = substituiertes oder unsubstituiertes C₁- bis C₁₂-Alkyl oder C₆- bis C₁₄-Aryl; und
- T =: O, NH oder NHR³,
wobei R³ = substituiertes oder unsubstituiertes C₁- bis C₁₂-Alkyl oder C₆- bis C₁₄-Aryl;
und
wobei der Molenbruch a der Einheit (IA), der Molenbruch b der Einheit (IB) und der Molenbruch c der Einheit (IC) wie folgt sind:
- a =: 0,05 bis 1,0;
- b =: 0 bis 0,95; und
- c =: 0 bis 0,90.

Bevorzugt ist das Polymer aus den Einheiten (IA) und gegebenenfalls (IB) und gegebenenfalls (IC) aufgebaut.

Weiter sind auch A-B und X in den einzelnen fünfgliedrigen Ringen unabhängig voneinander gewählt.

Die Alkyl- und Aryl-Gruppen von Z, R¹, R², R³, R⁴ und R⁵ können ggf. mit einer oder mehreren einfachen funktionellen Gruppen, insbesondere COOH, OH, C₁- bis C₆-Alkoxy oder Halogen, substituiert sein.

Zur Vereinfachung wird das erfindungsgemäße Polymer im folgenden durch die nachstehende allgemeine Formel (I) wiedergegeben:

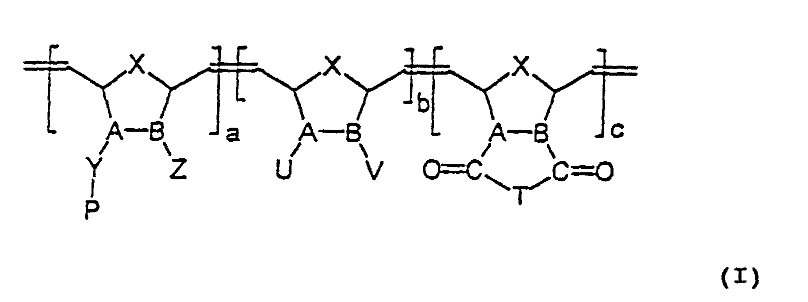

Der in Formel (I) gewählte Typ der vereinfachenden Darstellung wird in der Beschreibung und den Ansprüchen analog auch für andere Verbindungen verwendet.

Für die oben angegebenen Variablen des erfindungsgemäßen Polymers existieren unabhängig voneinander wählbare bevorzugte Definitionen, und diese sind wie folgt:
- X =: CH₂ oder O;
- A-B =: C-C;
- Y =: CH₂O oder CO-O-R¹-O;
- R¹ =: CH₂CH₂ oder CH₂-CHOH-CH₂;
- P =: CH₂=C(CH₃)-CO;
- Z =: H oder COOH;
- R⁴ =: CH₃, C₂H₅ oder Phenyl;
- U =: COOH;
- R⁵ =: CH₃, C₂H₅ oder Phenyl;
- V =: H oder COOH;
- R² =: CH₃ oder C₂H₅;
- T =: O;
- R³ =: CH₃ oder Phenyl;
- a =: 0,10 bis 0,80;
- b =: 0 bis 0,80; und/oder
- c =: 0 bis 0,60.

Bevorzugte Verbindungen sind demgemäß solche, bei denen mindestens eine der Variablen der Formel (I) die vorstehend beschriebende bevorzugte Definition aufweist.

Zur Herstellung des erfindungsgemäßen Polymers unterzieht man die bicyclische Verbindung (II) oder ggf. Mischungen von (II) mit der bicyclischen Verbindung (III) und/oder der bicyclischen Verbindung (IV) in Gegenwart eines Katalysators einer ringöffnenden Metathesepolymerisation und spaltet bei Einsatz von geschützten Edukten anschließend vorhandene Schutzgruppen ab. Dabei sind nach der Metathesepolymerisation die Positionen von A und B mit den daran gebundenen Resten nicht mehr voneinander unterscheidbar. Es können anstelle von (II), (III) und (IV) auch solche Verbindungen als Edukte eingesetzt werden, bei denen lediglich die Positionen von A und B mit den daran gebundenen Resten vertauscht sind.

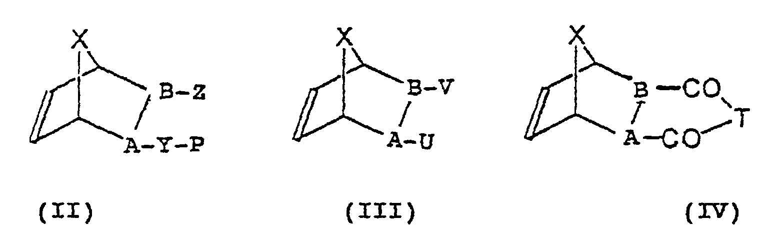

Beispiele für geeignete Schutzgruppen sind Trimethylsilyl- und Tetrahydropyranyl-Gruppen.

Darüber hinaus ist das erfindungsgemäße Polymer (I) auch durch polymeranaloge Umsetzung des Polymers (V) mit polymerisationsfähigen Edukten P-Halogen entsprechend nachstehender Reaktionsgleichung zugänglich:

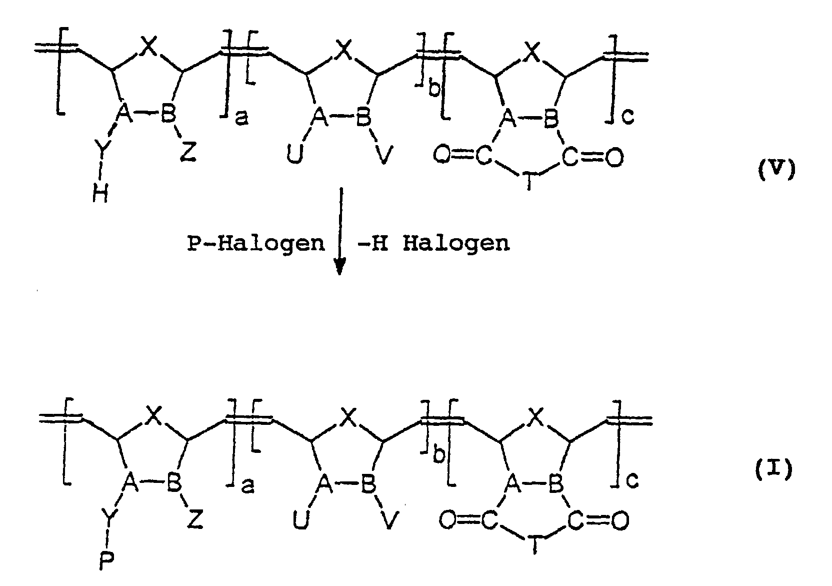

Das Polymer (V) kann durch ringöffnende Metathesepolymerisation einer Mischung der bicyclischen Verbindung (VI) mit der bicyclische Verbindung (III) und der bicyclische Verbindung (IV) in Gegenwart eines geeigneten Katalysators hergestellt werden.

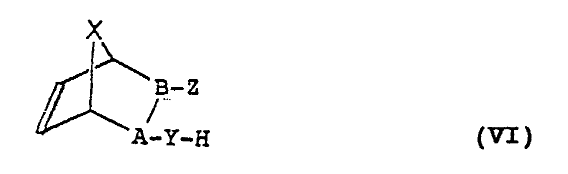

Als Katalysatoren für die ringöffnende Methathesepolymerisation (RÖMP) können bekanntlich Katalysator-Systeme auf der Basis von Verbindungen der Übergangsmetalle der IV. bis VIII. Nebengruppe eingesetzt werden (vgl. Encyclopedia of Polymer Science and Engineering, Vol. 9, J. Wiley & Sons, New York 1987, S. 648 ff.). Vor allem Verbindungen von Mo, W, Ru, Os und Ir finden Anwendung. Übliche Katalysatoren basieren auf Metallcarben-Komplexen, wie z.B. Ru-, W- oder Mo-Carben-Komplexe (vgl. R. R. Schrock, Acc. Chem. Res. **23** (1990) 158). Einfache Salze wie K₂RuCl₅ oder die Hydrate von RuCl₃ oder OsCl₃ eignen sich besonders für die ringöffnende Metathesepolymerisation von polaren Monomeren (W. J. Feast, D. B. Harrison, Polymer **32** (1991) 558). In Abhängigkeit vom verwendeten Katalysator können verschiedenartige Lösungsmittel eingesetzt werden. So finden z.B. im Falle von Metallcarben-Komplexen aprotische Lösungsmittel, wie THF, Benzol, Chlorbenzol, Toluol, Pentan oder Dichlormethan, Anwendung, während mit RuCl₃ die ringöffnende Metathesepolymerisation üblicherweise in wäßrig-alkoholischem Medium durchgeführt wird. Zur Regelung der Molmasse der erhaltenen Polymeren eignet sich besonders die Verwendung von 1-Alkenen, cis-2-Buten-1,4-diol oder Acrylsäure. Die Temperatur, bei der die Polymerisation durchgeführt wird, liegt bei der Verwendung von Metallcarbenen als Katalysator normalerweise im Bereich von 15-60 °C und im Falle von z.B. RuCl₃ bei 40 bis 70 °C.

Die als Edukte zur Herstellung des erfindungsgemäßen Polymers eingesetzten bicyclischen Verbindungen der Formeln (II), (III), (IV) und (VI) sind bekannt oder können nach bekannten Verfahren in einfacher Weise hergestellt werden.

Vertreter der allgemeinen Formel (II) sind z.B. Bicyclo[2.2.1]hept-5-en-2-ylmethyl-allylether (VII), Bicyclo[2.2.1.]hept-5-en-2-ylmethylacrylat (VIII), Bicyclo[2.2.1]hept-2,5-dien-2-ylmethylmethacrylat (IX), 7-Oxabicyclo[2.2.1]hept-5-en-2-ylmethylmethacrylat (X), Bicyclo[2.2.1]hept-5-en-2-ylmethylmethacrylat (XI) oder das 1:1-Umsetzungsprodukt (XII) von Bicyclo[2.2.1]hept-5-en-2,3-dicarbonsäureanhydrid und 2-Hydroxyethylmethacrylat, wobei (XI) und (XII) bekannt sind.

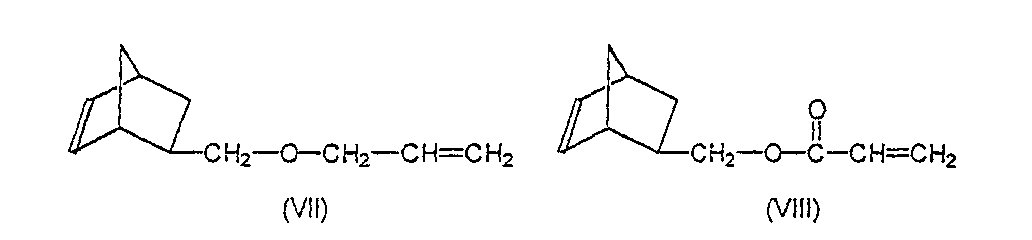

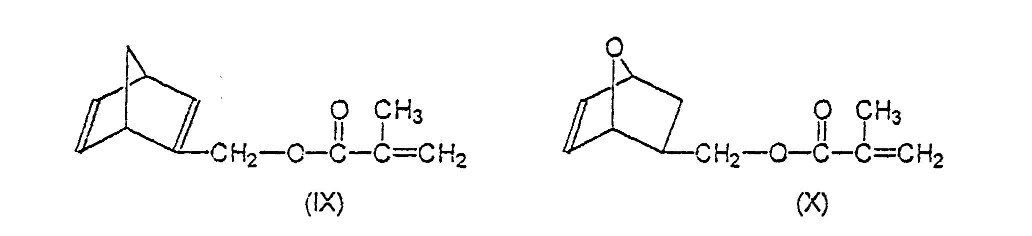

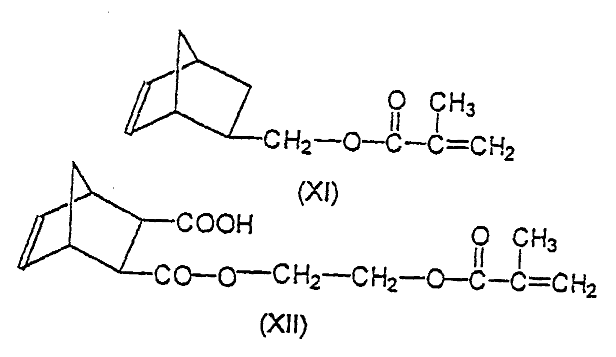

Vertreter der allgemeinen Formel (III) sind z.B. Bicyclo[2.2.1]hept-5-en-2-carbonsäure (XIII), Bicyclo[2.2.1]hept-5-en-2,3-dicarbonsäuremonomethylester (XIV), Bicyclo[2.2.1]hept-2,5-dien-2,3-dicarbonsäure (XV), Bicyclo[2.2.1]hept-5-en-2,3-dicarbonsäure (XVI) oder 7-Oxabicyclo[2.2.1]hept-5-en-2,3-dicarbonsäure (XVII), wobei (XVI) und (XVII) bekannt sind.

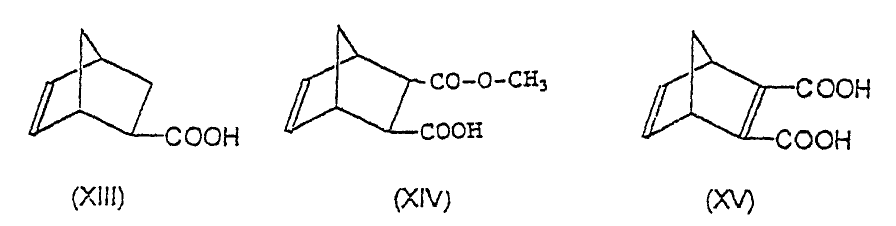

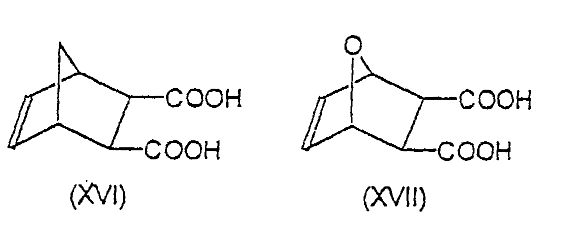

Vertreter der allgemeinen Formel (IV) sind z.B. Bicyclo[2.2.1]hept-5-en-2,3-dicarbonsäureimid (XVIII), Bicyclo[2.2.1]hept-2,5-dien-2,3-dicarbonsäureanhydrid (XIX), Bicyclo[2.2.1]hept-2,5-dien-2,3-dicarbonsäure-N-phenglimid (XX), Bicyclo[2.2.1]hept-5-en-2,3-dicarbonsäureanhydrid (XXI) oder 7-Oxabicyclo[2.2.1]hept-5-en-2,3-dicarbonsäureanhydrid (XXII), wobei (XXI) und (XXII) (vgl. O. Diels, K. Alder, Chem. Ber. **62,** (1929) S. 557) bekannt sind.

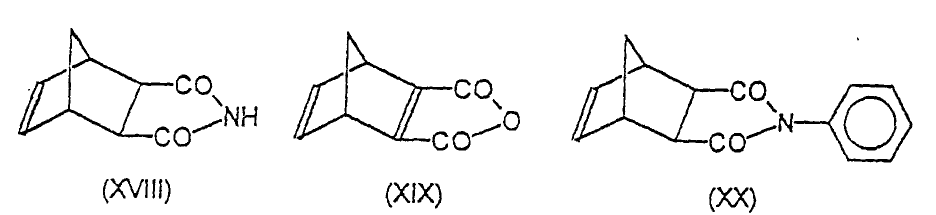

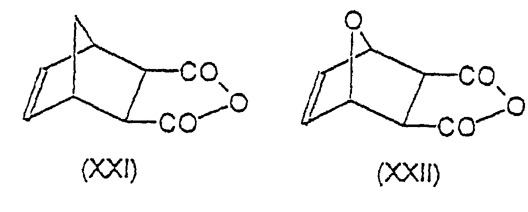

Vertreter der allgemeinen Formel (VI) sind z.B. 7-Oxabicyclo[2.2.1]hept-5-en-2-carbonsäure (XXIII), 7-Oxabicyclo[2.2.1]hept-2,5-dien-2-carbonsäure (XXIV), Bicyclo[2.2.1]hept-5-en-2-carbonsäure-2-hydroxyethylester (XXV), Bicyclo[2.2.1]hept-5-en-2-methanol (XXVI) oder Bicyclo[2.2.1]hegt-5-en-2-carbonsäure (XIII), wobei (XXVI) und (XIII) bekannt sind.

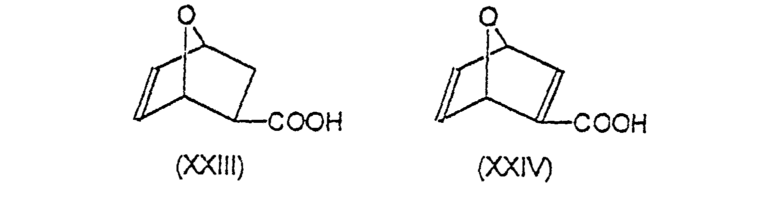

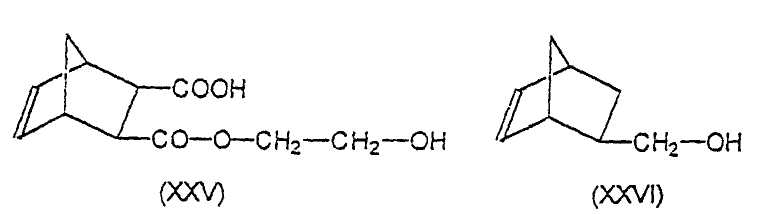

Die bicyclischen Verbindungen der Formeln (II) bis (IV) sowie solche der Formel (VI) lassen sich in einfacher Weise durch Diels-Alder-Reaktion (vgl. H. Wollweber, Diels-Alder-Reaktion, G. Thieme-Verlag 1972) von Cyclopentadien oder Furan mit geeigneten Dienophilen, wie Maleinsäure, Acetylendicarbonsäure oder Acrylsäuren oder deren Derivaten, und ggf. daran anchließende Modifizierung, wie z.B. durch Reduktion, Hydrolyse, Veretherung oder Veresterung, der erhaltenden bicyclischen Verbindungen, herstellen . So kann z.B. die Verbindung (VII) durch Diels-Alder-Reaktion von Cyclopentadien mit Acrylsäuremethylester, anschließende Reduktion des bicyclischen Adduktes zu 5-Norbornen-2-methanol (XXVI) und dessen Veretherung mit Allylbromid, synthetisiert werden.

Spezielle erfindungsgemäße Polymere lassen sich insbesondere durch ringöffnende Metathese-Copolymerisation von dem bekannten Bicyclo[2.2.1]hept-5-en-2-ylmethylmethacrylat (XI) mit einer trimethylsilyl- oder tetrahydropyranylgeschützten, käuflichen Bicyclo[2.2.1]hept-5-en-2,3-dicarbonsäure in Gegenwart eines geeigneten Molybdän-Carben-Katalysators und nachfolgende Entschützung durch saure Hydrolyse herstellen. Analog dazu können erfindungsgemäße Polymere auch durch Copolymerisation von 7-Oxabicyclo[2.2.1]hept-5-en-2-ylmethylmethacrylat (X) mit dem bekannten 7-Oxabicyclo[2.2.1]hept-5-en-2,3-dicarbonsäureanhydrid (XXII) in Gegenwart von Ruthenium(III)-chlorid in wäßrigalkohlischem Medium erhalten werden.

Das erfindungsgemäße Polymer eignet sich besonders als Bestandteile von Zementen, Beschichtungsmaterialien und Kompositen und insbesondere von Adhäsiven. Besonders bevorzugt wird das erfindungsgemäße Polymer als Dentalmaterial oder Bestandteil von Dentalmaterial, insbesondere als Bestandteil von Dentaladhäsiven, verwendet. Dabei erweist sich seine Fähigkeit als vorteilhaft, daß es einerseits über vorhandene polymerisierbare Gruppen, wie (Meth)acrylatgruppen einen Verbund mit dem zu befestigenden Material, z.B. einem Kompositmaterial, bildet und andererseits über Carboxylgruppen eine die Haftung fördernde Wechselwirkung mit dem Substrat, wie insbesondere der Zahnhartsubstanz, ausbilden kann.

Bei Verwendung des erfindungsgemäßen Polymers als Bestandteil von Dentalmaterialien wird es üblicherweise in einer Menge von 0,1 bis 60, insbesondere 1,0 bis 40 Gew.-%, bezogen auf das Dentalmaterial, eingesetzt. Zur Herstellung der Dentalmaterialien wird das erfindungsgemäße Polymer insbesondere mit polymerisierbaren organischen Bindemitteln, Vernetzermonomeren, Füllstoffen, reaktiven Füllstoffen, Polymerisationsinitiatoren und/oder weiteren Zusätzen, wie üblichen Stabilisatoren, z.B. Hydrochinonmonomethylether (MEHQ) oder 2,6-Di-tert.-butyl-4-methylphenol (BHT), UV-Absorbern, Pigmenten, Farbstoffen oder Lösungsmitteln, kombiniert.

Als polymerisierbare organische Bindemittel eignen sich alle für einen Dentalwerkstoff brauchbaren Bindemittel, insbesondere monofunktionelle oder polyfunktionelle (Meth)acrylate, die allein oder in Mischungen eingesetzt werden können. Bevorzugte Beispiele für diese Verbindungen sind Methyl(meth)acrylat, Isobutyl(meth)acrylat, Cyclohexyl(meth)acrylat, Tetraethylenglycoldi(meth)acrylat, Triethylenglycoldi(meth)acrylat, Diethylenglycoldi(meth)-acrylat, Ethylenglycoldi(meth)acrylat, Polyethylenglycoldi(meth)acrylat, Butandioldi(meth)acrylat, Hexandioldi(meth)acrylat, Decandioldi(meth)acrylat, Dodecandioldi(meth)acrylat, Bisphenol-A-di(meth)acrylat, Trimethylolpropantri(meth)acrylat, 2,2-Bis-4-(3-methacryloxy-2-hydroxy-propoxy)-phenylpropan (Bis-GMA) sowie die Produkte der Reaktion von Isocyanaten, insbesondere Di- und/oder Triisocyanaten, mit OH-gruppenhaltigen (Meth)acrylaten. Besonders bevorzugte Beispiele für die zuletzt genannten Produkte sind durch Reaktion von 1 Mol Hexamethylendiisocyanat mit 2 Mol 2-Hydroxyethylenmethacrylat, von 1 Mol Tri-(6-isocyanatohexyl)biuret mit 3 Mol 2-Hydroxyethylmethacrylat und von 1 Mol 2,2,4-Trimethylhexamethylendiisocyanat mit 2 Mol 2-Hydroxyethylmethacrylat erhältlich.

Die organischen Bindemittel werden üblicherweise in einer Menge von 0 bis 90 Gew.-% in dem erfindungsgemäßen Dentalmaterial eingesetzt.

Als Vernetzermonomere eignen sich insbesondere die vorstehend genannten polyfunktionellen (Meth)acrylate, insbesondere Triethylenglycoldi(meth)acrylat, Polyethylenglycoldi(meth)acrylat, Bis-GMA oder Urethandi(meth)acrylate.

Die Vernetzermonomere werden üblicherweise in einer Menge von 0 bis 80 Gew.-% in dem erfindungsgemäßen Dentalmaterial eingesetzt .

Beispiele für bevorzugte Füllstoffe sind Quarz-, Glaskeramik- und Glaspulver, insbesondere Bariumsilikatgläser, Li/Al-Silikatgläser und Bariumgläser, Aluminium- oder Siliciumoxide, feinstteilige Kieselsäuren, insbesondere pyrogene oder gefällte Kieselsäuren, röntgenopake Füllstoffe, wie Ytterbiumtrifluorid.

Die Füllstoffe werden typischerweise in einer Menge von 0 bis 85 Gew.-%, bezogen auf das Dentalmaterial, eingesetzt.

Als reaktive Füllstoffe kommen vor allem Fluoralumosilicat-Gläser und andere bei Glasionomer-Zementen eingesetzte Gläser in Frage. Die reaktiven Füllstoffe werden üblicherweise in einer Menge von 0 bis 80 Gew.-% in dem erfindungsgemäßen Dentalmaterial verwendet.

Besonders bevorzugte erfindungsgemäße Dentalmaterialien sind lichthärtende Glasionomerzemente, Dentinadhäsive und Compomere.

Ganz besonders vorteilhafte Glasionomerzemente, Dentinadhäsive und Compomere und deren jeweilige Komponenten sind nachstehend angegeben:

### Lichthärtende Glasionomerzemente:

- erfindungsgemäßes Polymer,
- reaktives Glaspulver, insbesondere übliche Fluoralumosilicatgläser einer mittleren Teilchengröße von ca. 0,05 bis 15µm (vgl. A. D. Wilson, J. W. McLean, Glasionomerzement, Quintessenz Verlags-GmbH, Berlin 1.988, S. 21 ff.),
- Polymere mit Carboxylgruppen, z.B. Acrylsäure oder Maleinsäure-Polymere, die ggf. seitenständig gebundene polymerisationsfähige Gruppen, z.B. (Meth)acrylgruppen tragen,
- Photoinitiatoren und Stabilistoren,
- H₂O, und
- Vernetzermonomere.

### Dentinadhäsive:

- erfindungsgemäßes Polymer,
- Hydrophile Monomere, wie 2-Hydroxyethyl- oder 2-Hydroxypropyl(meth)acrylat oder Polyethylenglycolmonooder -dimethacrylate oder n-Vinylpyrrolidon,
- polymerisationsfähige Carbonsäuren oder Phosphorsäuren, wie z.B. Maleinsäure oder 2-(Meth)acryloyloxyethyldihydrogenphosphat,
- Vernetzermonomere,
- Wasser, Alkohol oder Aceton und
- Photoinitiatoren und Stabilisatoren.

### Compomere:

- erfindungsgemäßes Polymer,
- Glaspulver, insbesondere für Glasionomerzemente übliche Fluoralumosilicatgläser einer mittleren Partikelgröße von ca. 0,05 bis 5 µm (vgl. A. D. Wilson, J. W. McLean, Glasionomerzement, Quintessenz Verlags-GmbH, Berlin 1988, S. 21 ff.),
- Photoinitiatoren und Stabilisatoren,
- übliche Vernetzermonomere,
- carboxylgruppenhaltige Venetzermonomere, wie die Umsetzungsprodukte von 2 Mol 2-Hydroxyethyl(meth)-acrylat oder 2-Hydroxypropyl(meth)acrylat mit 1 Mol 5-(2,5-Dioxotetrahydrofuryl)-3-methylcyclohex-3-en-1,2-dicarbonsäureanhydrid oder den Dianhydriden von z.B. der käuflichen Tetrahydrofuran-2,3,4,5-tetracarbonsäure, der Cyclohexan-1,2,3,4,5,6-hexacarbonsäure oder der Butan-1,2,3,4-tetracarbonsäure, sowie entsprechende Umsetzungsprodukte dieser multifunktionellen Carbonsäuren mit mehr als 1 Mol Glycidylmethacrylat.

Die erfindungsgemäßen Dentalmaterialien und die erfindungsgemäßen Polymere können heiß, kalt oder durch Licht polymerisiert werden. Als Initiatoren für die Heißpolymerisation können die bekannten Peroxide wie Dibenzoylperoxid, Dilauroylperoxid, tert.-Butylperoctoat oder tert.-Butylperbenzoat eingesetzt werden. Darüber hinaus sind auch 2,2'-Azoisobuttersäurenitril (AIBN), Benzpinakol und 2,2'-Dialkylbenzpinakole geeignet.

Als Initiatoren für die Photopolymerisation können zum Beispiel Benzophenon und seine Derivate sowie Benzoin und seine Derivate verwendet werden. Weitere bevorzugte Photoinitiatoren sind die α-Diketone wie 9,10-Phenanthrenchinon, Diacetyl, Furil, Anisil, 4,4'-Dichlorbenzil und 4,4'-Dialkoxybenzil. Besonders bevorzugt wird Campherchinon verwendet . Darüber hinaus eignet sich auch die Gruppe der Acylphosphinoxide gut zur Initiierung der Photopolymerisation. Zur Beschleunigung der Initiierung werden die Photoinitiatoren vorzugsweise zusammen mit einem Reduktionsmittel, besonders bevorzugt mit einem Amin, insbesondere einem aromatischen Amin, eingesetzt.

Als Initiatoren für die Kaltpolymerisation werden Radikale liefernde Redox-Systeme, zum Beispiel Benzoyl- oder Lauroylperoxid zusammen mit Aminen wie N,N-Dimethyl-p-toluidin, N,N-Dihydroxyethyl-p-toluidin oder anderen strukturverwandten Aminen eingesetzt.

Speziell bei Dentalmaterialien zur Zementierung von Dentalrestaurationen, wie Glaskeramik-Inlays, -Onlays, -Teilkronen und - Kronen, hat sich die Kombination von Photoinitiatoren mit unterschiedlichen Redoxsystemen bewährt. Bevorzugt sind Kombinationen aus Campherchinon, Benzoylperoxid und Aminen wie N,N-Dimethyl-ptoluidin und/oder N,N-Cyanoethylmethylanilin.

Die Konzentration der Initiatoren liegt bevorzugt im Bereich von 0,05 bis 2,0 Gew.-%, besonders bevorzugt im Bereich von 0,1 bis 0,8 Gew.-%, bezogen auf das Dentalmaterial.

Im folgenden wird die Erfindung anhand von Beispielen näher erläutert.

### Beispiele

### Beispiel 1: Methacrylsäure-(5-norbornen-2-endo/exo-methyl)ester (1)

In einem 1500 ml Sulfierkolben mit mechanischem Rührwerk, 150 ml Tropftrichter und Thermometer werden 31,7 g (0,25 Mol) 2-(Hydroxymethyl)-5-norbornen (Gemisch von endo/exo-Isomeren), 400 ml getrocknetes Tetrahydrofuran (THF), 26,8 g (0,27 Mol) frisch destilliertes Triethylamin (TEA) und 0,01 g 2,6-Di.-tert.butyl-cresol (BHT) bei 0°C unter Rühren und Argon-Schutzgasatmosphäre vorgelegt. Dazu tropft man eine Lösung von 29,5 g (0,28 Mol) Methacrylsäurechlorid in 100 ml THF so langsam zu, daß die Temperatur zwischen 0°C und 5°C bleibt. Dann läßt man das Reaktionsgemisch unter weiterem Rühren innerhalb von 60 Minuten auf Raumtemperatur kommen. Der Ansatz wird nun über eine Nutsche filtriert, und der abfiltrierte Rückstand wird mit 150-200 ml Diethylether gewaschen. Das Filtrat wird mit 150 ml einer gesättigten NaCl-Lösung extrahiert, die mit konzentrierter HCl auf pH 1-2 eingestellt ist. Dann werden die vereinigten organischen Phasen mit 2 × 100 ml gesättigter NaCl-Lösung neutral gewaschen und nachfolgend mit 150 ml einer gesättigten NaCl-Lösung extrahiert, die mit 50 ml 10%-iger Na₂CO₃-Lösung basisch eingestellt ist. Abschließend wird die organische Phase mit 2 × 100 ml gesättigter NaCl-Lösung neutral gewaschen, mit 50 g wasserfreiem Na₂SO₄ unter Rühren 20 Minuten lang getrocknet, filtriert und am Rotationsverdampfer bei 30°C eingeengt. Der erhaltene Rückstand wird im Feinvakuum (0,02 mbar) bei einer Badtemperatur von ca. 110°C innerhalb von 35 Minuten destilliert, wobei 23,5 g (55 % Ausbeute) einer farblosen Flüssigkeit (Kp_{0,02mbar} = 70-74 °C) erhalten werden.

| | | | | |
|---|---|---|---|---|
| Elementaranalyse: | C₁₂H₁₆O₂: | Ber.: | C 74,96 | H 8,39 |
| | (192,25) | Gef.: | C 74,44 | H 8,33 |

- ¹H-NMR (90MHz, CDCl₃):: 0,56 und 0,72 (m, 1H, CH-CH₂O); 1,25 1,60 (m, 2H, CH₂-CH-CH₂O); 1,70-1,95 und 2,41-2,7 (m, 2 × 1H, CH-norbornen); 2,00 (s, 3H, CH₃); 2,75-3,0 (b, 2H, CH₂-norbornen); 3,65-4,30 (m, 2H, CH₂O); 5,60 und 6,18 (s, 2 × 1H, CH₂=,); 5,95-6,14 und 6,17-6,30 (m, 2 × 1H, CH=CH, von CH₂= überlagert)
- IR (Film, cm⁻¹) :: 1719(C=O); 1636(C=C)

### Beispiel 2: 7-Oxabicyclo[2.2.1]hept-5-en-2,3-endo/exo-dicarbonsäurebis(trimethylsilyl)ether (2)

In einem 500 ml Sulfierkolben mit mechanischem Rührwerk, Tropftrichter und Thermometer werden 14,8 g (0,1 Mol) 7-Oxabicyclo[2.2.1]hept-5-en-2,3-endo/exo-dicarbonsäure in 150 ml getrocknetem THF gelöst. Die Apparatur wird mit Argon gespült, und die vorgelegte Mischung wird unter Rühren auf 10-15°C abgekühlt. Es werden dann 20,2 g (0,2 Mol) TEA und 21,7 g (0,2 Mol) Trimethylsilylchlorid zugetropft. Zur Vervollständigung der Reaktion rührt man noch 3 Stunden lang nach. Der gebildete Niederschlag aus TEA-Hydrochlorid wird unter Argon abgesaugt, mit THF gewaschen, und die vereinigten organischen Phasen werden am Rotationsverdampfer bei 30-40 °C eingeengt. Nach dem Trocknen im Feinvakuum erhält man ca. 32 g eines farblosen Öls, das nach Abkühlen im Tiefkühlschrank fest wird. Zur Reinigung wird aus 900 ml wasserfreiem Petrolether umkristallisiert und im Feinvakuum getrocknet. Es werden 18,6 g (58 % Ausbeute) farblose Kristalle (Schmp.: 65-70 °C) erhalten.

| | | | | |
|---|---|---|---|---|
| Elementaranalyse: | C₁₄H₂₄O₅Si₂ | Ber.: | C 51,18 | H 7,36 |
| | (328,50) | Gef.: | C 50,90 | H 6,18 |

- ¹H-NMR (90 MHz, CDCl₃) :: 0,26 ( s, 18H, CH₃Si); 2,78 (d, 3H, CHCOO-exo); 3,34 (m, 7H, CHCOO-endo); 5,10 (b, 7H, CHOCH-endo); 5,22 (b, 3H, CHOCH-exo); 6,48 (s, 3H, CH=CH-exo); 6,55 (s, 7H, CH=CH-endo),
- ¹³C-NMR (75 MHz, CDCl₃):: 0,02 (CH₃ ↑); 47,7 und 49,5 (CH-C=O ↑); 80,4 (C-O-C ↑); 135,1 und 135,5 (C=C ↑); 170,8 und 175,3 (C=O (-)).

### Beispiel 3: Bicyclo[2.2.1]hept-5-en-2,3-endo/exo-dicarbonsäurebis(tetrahydropyran-2-yl)ester (3)

Zu einer Lösung von 18,2 g (0,10 Mol) 5-Norbornen-2,3-endo/exo-dicarbonsäure und 1,5 g (6 mMol) Pyridiniumtosylat in 100 ml absolutem Dichlormethan werden 21,2 g (0,25 Mol) 3,4-Dihydro-2Hpyran (DHP) bei Raumtemperatur zugegeben, und die Mischung wird 42 Stunden lang gerührt. Die gebildete gelblich gefärbte Lösung wird mit 2 × 50 ml gesättigter NaHCO₃-Lösung ausgeschüttelt. Die erhaltene organische Phase wird über wasserfreiem Na₂SO₄ getrocknet und durch eine Schicht aus basischem Al₂O₃ abgesaugt. Schließlich werden das Lösungsmittel und überschüssiges DHP im Rotationsverdampfer abgezogen. Das verbleibende Öl wird in 200 ml Hexan bei 50 °C gelöst und nochmals durch eine Schicht aus basischem Al₂O₃ filtriert. Nach Einengen der filtrierten Hexan-Lösung auf ca. 70 ml und Akühlen fallen ca. 25 g (71 % Ausbeute) farblose Kristalle aus (Schmp.: 82-96 °C). 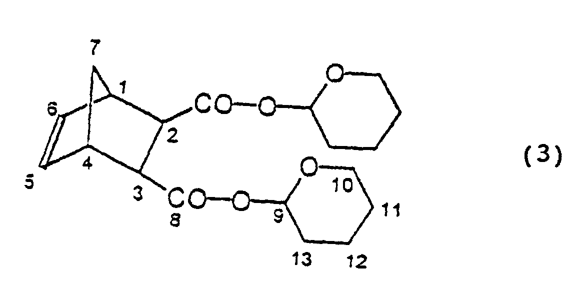
- ¹H-NMR (300MHz, CDCl₃) :: 1,40-1,90 (14H, m, H7, H11, H12, H13), 2,26 (1H, dd, H2), 3,20 (1H, br. s, H1), 3,33 (1H, br. s, H4), 3,46 (1H, m, H3), 3,70 (2H, m, H10), 3,85 (2H, m, H10), 5,95 (1H, br. s, H9), 6,02 (1H, br. s, H9), 6,13 (1H, m, H5) und 6,33 (1H, dd, H6)
- ¹³C-NMR (75 MHz, CDCl₃):: 18,6, 25,2, 25,5, 31,3 (C11, C12, C13), 46,0 (C4), 47,0-48,4 (C1, C2, C3, C7), 63,1 (C10), 93,0 (C9), 135,2 (C5), 137,6 (C6), 171,8, 173,1 und 179,3 (C=O)

### Beispiel 4: Synthese eines als Katalysator verwendbaren Molybdän-Carben-Komplexes

### (Analog: R. R. Schrock et al., J. Amer. Chem. Soc. 112, (1990) 3875)

Der in diesem Beispiel beschriebene Katalysator besitzt die nachstehende Strukturformel und wird im folgenden als Katalysator **HF** bezeichnet.

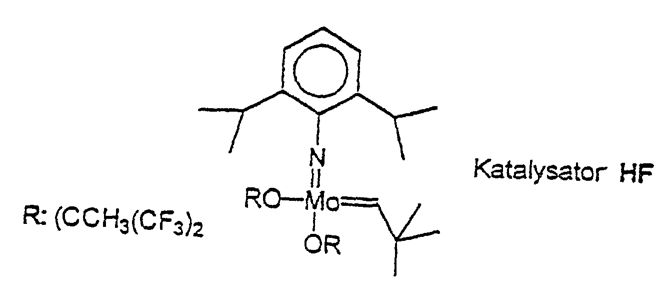

2,22 g (11,8 mMol) Lithium-tert.-1,1,1,3,3,3-hexafluorbutoxid werden als Feststoff langsam bei -30 °C zu einer Lösung von 4,0 g (5,5 mMol) Mo(CHCMe₃)(NAr)(OSO₂CF₃)₂(dme) (Ar = 2,6-Diisopropylphenyl, dme = Ethylenglycoldimethylether) in einer Mischung aus 200 ml Diethylether und 200 ml Ethylenglycoldimethylether innerhalb von 10 Minuten gegeben. Die Reaktionsmischung wird anschließend auf Raumtemperatur gebracht, 2 Stunden lang gerührt und bis zur Trockene eingeengt. Der dunkelorange Feststoff wird mit ca. 50 ml Pentan extrahiert, durch eine Schicht aus Celite® filtriert und eingeengt. Der Rückstand wird aus Pentan umkristallisiert. Der erhaltene Katalysator HF kann für die ringöffnende Metathesepolymerisation eingesetzt werden.
- ¹H-NMR (300 MHz, CDCl₃) :: 1,04 (s, 1H, MOCHCMe₃), 1,16 (d, 12H, CH(CH₃)₂), 1,37 (s, 6H, OCMe(CF₃)₂), 3,54 (sept, 2H, CHMe₂), 6,94 (m, 3H, NAr) und 12,06 (s, 1H, MoCHCMe₃)

### Beispiel 5:

### (A:) Allgemeine Vorschrift für ringöffnende Metathesepolymerisation mit Metallcarben-Katalysatoren zur Synthese der erfindungsgemäßen Polymeren:

Die ringöffnende Metathesepolymerisation mit Übergangsmetallcarben-Komplexen wird unter Inertgasatmosphäre in absoluten aprotischen Lösungsmitteln, wie Chlorbenzol, Benzol, Toluol, THF oder Dichlormethan, durchgeführt, wobei 1-Alkene, z.B. 1-Hexen, als Kettenüberträger zur Einstellung der Molmasse verwendet werden.

So werden unter Rühren zu einer 10-20 Gew.-%-igen Lösung von 100-1000 Gew.-Teilen Monomeren 1-25 Gew.-Teile Kettenüberträger und eine 10 Gew.-%-ige Lösung von 1 Gew.-Teil Katalysator zugegeben. Man polymerisiert bei Raumtemperatur. Nach Beendigung der Polymerisation wird mit Benzaldehyd abgebrochen, und das Polymer wird in Methanol ausgefällt und durch Umfällen gereinigt.

### (B:) Synthese des erfindungsgemäßen Copolymers (4)

8,00 g (22,8 mMol) Monomer (3) (vgl. Beispiel 3) und 1,10 g (5,7 mMol) Monomer (1) (vgl. Beispiel 1) werden unter Schutzgas zusammen mit 25 mg (0,30 Mol) 1-Hexen in 40 ml Chlorbenzol gelöst. Zu dieser Lösung wird unter Rühren eine Lösung von 30 mg (0,043 mMol) Katalysator HF gegeben. Nach 4 Stunden wird die Polymerisation mit ca. 0,3 ml Benzaldehyd abgebrochen, und das erhaltene Polymer wird in 300 ml mit BHT (100 ppm) stabilisiertem Ethanol ausgefällt und anschließend getrocknet. Zur Abspaltung der THP-Schutzgruppe wird das Polymer in THF gelöst, und die gebildete Lösung wird in eine heiße Lösung von ca. 50 mg p-Toluolsulfonsäure in 30 ml stabilisiertem Ethanol so langsam eingetropft, daß sich die jeweils entstehende Trübung auflöst. Man rührt noch 20 Minuten lang und fällt das in Ethanol lösliche Polymer (4) aus einem Gemisch aus Pentan/Diethylether (2/1-Volumenteile) aus. Das erhaltene Polymer wird schließlich unter Lichtschutz im Feinvakuum getrocknet, und sein zahlenmittleres Molekulargewicht wird zu (Mₙ = 13500 g/Mol) bestimmt. Die Molekulargewichtsbestimmung erfolgte mittels Gelpermeationschromatographie (GPC) bei Kalibrierung mit Polystyrolstandards.

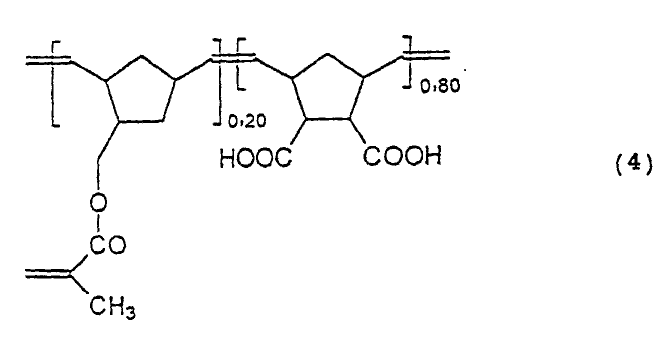

### Beispiel 6: Synthese des erfindungsgemäßen Terpolymers (5)

Analog zu Beispiel 5 wird zu einer Lösung von 2,63 g (7,5 mMol) Monomer (3), 0,29 g (1,5 mMol) Monomer (1), 0,24 g (1,0 mMol) Bicyclo[2.2.1]hept-5-en-2,3-exo/endo-dicarbonsäure-N-phenylimid, das einfach durch Diels-Alder-Reaktion von Cyclopentadien und N-Phenylmaleinimid zugänglich ist, und 6,1 mg (0,072 mMol) 1-Hexen in 8 ml Methylenchlorid eine Lösung von 23,2 mg (0,033 mMol) Katalysator HF in 2 ml Methylenchlorid zugegeben. Die Polymerisationslösung wird rasch hochviskos. Nach 2 Stunden wird die Polymerisation durch Eintropfen der Polymerisationslösung in ca. 100 ml Ethanol abgebrochen. Das ausgefallene Polymer wird abfiltriert und getrocknet und zeigt eine zahlenmittlere Molmasse (GPC, Kalibrierung mit Polystyrolstandards) von 31000 g/Mol. Zur Abspaltung der THP-Schutzgruppe wird das Polymer in 10 ml THF gelöst und die Lösung in eine 50°C warme Lösung von 30 mg p-Toluolsulfonsäure in 50 ml Ethanol so langsam eingetropft, daß sich der gebildete Niederschlag immer wieder auflöst. 1,3 g (68 % Ausbeute) an Terpolymer (5) werden schließlich durch Eintropfen der erhaltenen Polymerlösung in 400 ml Diethylether und Trocknen des erhaltenen Rückstandes im Vakuum erhalten. Die mittels GPC bestimmte zahlenmittlere Molmasse beträgt 14000 g/Mol, wobei das ¹H-NMR-Spektrum zeigt, daß die Zusammensetzung des Terpolymers der Zusammensetzung der eingesetzten Monomer-Mischung entspricht.

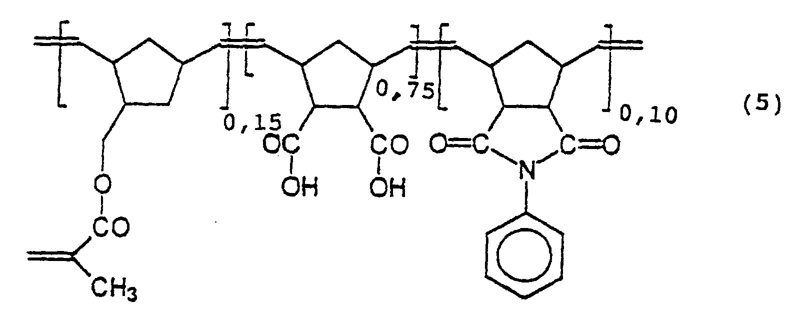

### Beispiel 7: Synthese erfindungsgemäßer Polymere durch ringöffnende Metathesepolymerisation von Monomer (3) und nachfolgende polymeranaloge Reaktion mit GMA

### Ringöffnende Metathesepolymerisation:

Analog zu Beispiel 5 wird zu einer Lösung von 23 g Monomer (3) und 153 mg 1-Hexen in 80 ml Chlorbenzol eine Lösung von 50 mg Katalysator HF in 2 ml Chlorbenzol gegeben. Nach 2 Stunden wird nochmals die gleiche Menge an Katalysatorlösung hinzugefügt. Nach 4 Stunden wird die Polymerisation abgebrochen. Die Polymerisationslösung wird mit 300 ml Ethanol verdünnt und nach Zugabe von 50 mg p-Toluolsulfonsäure zur Abspaltung der Schutzgruppe 30 Minuten lang unter Rückfluß erhitzt. Die erhaltene Lösung wird dann im Vakuum nahezu bis zur Trockene eingeengt. Der entstandene Rückstand wird in THF gelöst, und die THF-Lösung wird in 200 ml Diethylether eingetropft, woraufhin ein Niederschlag ausfällt. Nach Abtrennung und Trocknen im Feinvakuum werden ca. 11 g (Ausbeute 92 %) Polymer (**6**) mit einer zahlenmittleren Molmasse von 1200 g/Mol (Gelpermationschromatographie (GPC) - mit Polystyrolstandard) erhalten.

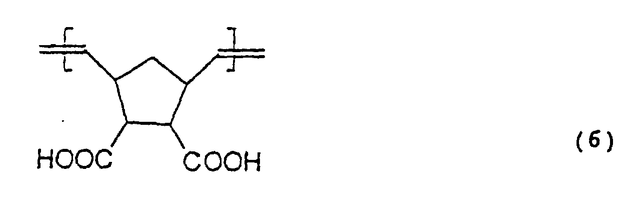

### Polymeranaloge Umsetzung mit Glycidylmethacrylat (GMA) :

Zu einer Lösung von 5 g (27,2 mMol) Polymer (**6**) in 20 ml sec. Butanol werden 1,55 g (10,9 mMol) GMA, 11,6 mg (0,11 mMol) Lithiumperchlorat als Katalysator und eine Spatelspitze Hydrochinonmonomethylether (MeHQ) gegeben. Die Reaktionsmischung wird 24 Stunden lang bei 60 °C gerührt. Das Reaktionsprodukt wird dann durch Eingabe der Mischung in 150 ml Ethylacetat ausgefällt, in Methanol gelöst und durch Eintropfen der erhaltenen Lösung in Ethylacetat erneut ausgefällt und schließlich im Feinvakuum getrocknet. Es werden 3,8 g Polymer (**7**) erhalten, wobei die Struktur mittels Titration der freien Carboxylgruppen und ¹H-NMRspektroskopisch bestätigt wurde.

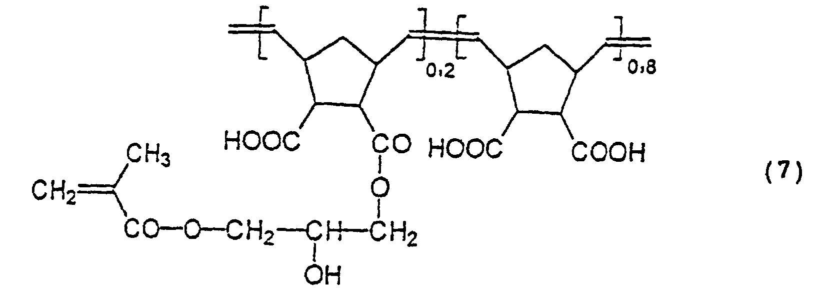

### Beispiel 8: Dentinadhäsiv auf der Basis vom Copolymer (4)

Auf der Basis von Copolymer (**4**) wird ein 1-Komponenten-Dentinadhäsiv folgender Zusammensetzung hergestellt:

| | |
|---|---|
| Copolymer (**4**) | 18,0 Gew.-% |
| Wasser, deionisiert | 32,4 Gew.-% |
| 2-Hydroxyethylmethacrylat | 44,2 Gew.-% |
| Maleinsäure | 3,0 Gew.-% |
| Campherchinon | 0,3 Gew.-% |
| MeHQ | 0,1 Gew.-% |
| Ammoniumfluorid | 1,0 Gew.-% |
| Diphenyliodoniumhexafluorophosphat | 1,0 Gew.-% |

Zur Ermittlung der mit diesem Dentinadhäsiv erzielbaren Scherhaftung wurden zuerst Dentinoberflächen von extrahierten, eingebetteten Zähnen mit 500-er und 1000-er Schleifpapier plangeschliffen. Dann wurden die Dentinoberflächen mit Zellstoff leicht getrocknet. Auf die Oberflächen wurde das Dentinadhäsiv in 2 Schichten aufgetragen, mit Druckluft getrocknet und mit einer dentalen Lichtquelle Heliolux GTE (Fa. Vivadent) belichtet. Dann wurde ein kommerziell erhältliches Füllungs-Komposit, nämlich Compoglass der Firma Vivadent, Liechtenstein, in 2 Schichten aufgetragen und jeweils 40 Sekunden lang belichtet. Danach wurden die erhaltenen Prüfkörper in destilliertes Wasser gelegt und dort 24 Stunden lang bei 37 °C gelagert. Die Scherhaftfestigkeit wurde schließlich gemäß ISO-Vorschlag ISO-TR 11405: "Dental material - Guidance on testing of adhesion to tooth structure", zu 15,6 ± 7,8 MPa bestimmt. Eine analoge Rezeptur, bei der das Polymer (4) durch herkömmliche Polyacrylsäure, nämlich Plex 4779 der Fa. Röhm ersetzt wurde, ergab nur Werte von 3,9 ± 0,6 MPa.

### Beispiel 9: Lichthärtender Glasionomer-Zement auf der Basis vom Copolymer (4)

| Flüssigkeit 1: | |
|---|---|
| Urethandimethacrylat¹⁾ | 56,5 Gew.-% |
| 2-Hydroxyethylmethacrylat | 18,8 Gew.-% |
| PEG-600-Dimethacrylat | 18,8 Gew.-% |
| 1-%ige wäßrige Lösung von p-Toluolsulfonsäure | 5,1 Gew.-% |
| 2-Cyanoethylmethylanilin | 0,5 Gew.-% |
| Campherchinon | 0,3 Gew.-% |

| Pulver 2: | |
|---|---|
| Reaktives Glaspulver²⁾ | 92,5 Gew.-% |
| Copolymer (**4**) | 6,0 Gew.-% |
| L(+)-Weinsäure | 1,5 Gew.-% |

| | |
|---|---|
| ¹⁾ Urethandimethacrylat aus 2 Mol 2-Hydroxyethylmethacrylat und 1 Mol 2,2,4-Trimethylhexamethylendüsocyanat-1,6. | |
| ²⁾ Glaszusammensetzung (gemäß Atomabsorptionsspektrometrie und Titration für Fluorid-Bestimmung) (Gew.-%): SiO₂ : 25,5, Al₂O₃: 23,8, Na₂O: 1,8, CaO: 16,4, P₂O₅: 8,2, BaO: 11,2 und Fluorid: 17,0; mittlere Korngröße 8 µm. | |

Aus jeweils einem Gewichtsteil der Flüssigkeit 1 und des Pulvers 2 wird eine Mischung hergestellt. Mit dem Material werden Prüfkörper hergestellt, die 2 Stunden lang bei 37 °C in H₂O gelagert und anschließend zweimal 90 Sekunden lang mittels einer dentalen Strahlungsquelle, Spectramat (Fa. Vivadent), bestrahlt werden. Die nach der ISO-Norm 9917 (Dental water-based cements) bestimmte Druckfestigkeit beträgt 128,4 MPa, im Vergleich zu 117,1 MPa für ein Material, bei dem das Copolymer (**4**) im Pulver durch den gleichen Anteil an Polyacrylsäure Plex 4779 ersetzt wurde.

## Patentansprüche

1. Funktionalisiertes und polymerisierbares Polymer, **dadurch gekennzeichnet, daß** es die folgenden wiederkehrenden Einheiten (IA), (IB) und (IC) aufweist: 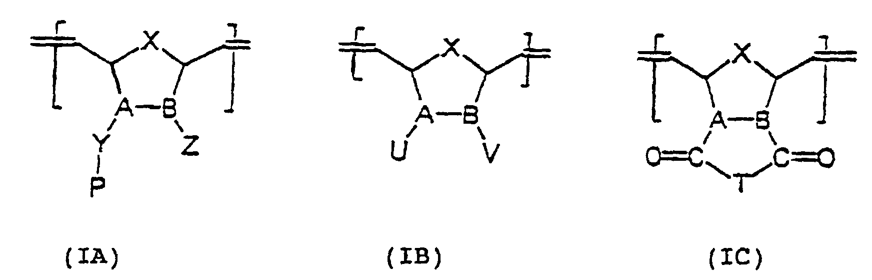 wobei X, A-B, Y, P, Z, U, V, T, R¹, R², R³, R⁴ und R⁵ unabhängig voneinander die folgenden Bedeutungen haben:
X = CH₂ oder O;
A-B = C-C oder C=C;
Y = CH₂O, CO-O oder COO-R¹-O,
wobei R¹ = substituiertes oder unsubstituiertes C₁ - bis C₅-Alkylen oder -Oxyalkylen;
P = eine polymerisierbare Gruppe, nämlich CH₂=CH-CO-, CH₂=C(CH₃)-CO-, CH₂=CH-CH₂- oder CH₂=CH-C₆H₅-CH₂-;
Z = H, COOH, substituiertes oder unsubstituiertes C₁- bis C₁₂-Alkyl oder COOR⁴,
wobei R⁴ = substituiertes oder unsubstituiertes C₁- bis C₁₂-Alkyl oder C₆- bis C₁₄-Aryl;
U = COOH oder COOR⁵,
wobei R⁵ = substituiertes oder unsubstituiertes C₁ - bis C₁₂-Alkyl oder C₆- bis C₁₄-Aryl;
V = H, COOH, CH₂-OH, OR² oder CO-OR²,
wobei R² = substituiertes oder unsubstituiertes C₁- bis C₁₂-Alkyl oder C₆- bis C₁₄-Aryl; und
T = O, NH oder NHR³,
wobei R³ = substituiertes oder unsubstituiertes C₁bis C₁₂-Alkyl oder C₆- bis C₁₄-Aryl;
und
wobei der Molenbruch a der Einheit (IA), der Molenbruch b der Einheit (IB) und der Molenbruch c der Einheit (IC) wie folgt sind:
a = 0,05 bis 1,0;
b = 0 bis 0,95; und
c = 0 bis 0,90.

2. Polymer nach Anspruch 1, **dadurch gekennzeichnet, daß** die Variablen unabhängig voneinander die folgende Bedeutung haben:
X = CH₂ oder O;
A-B = C-C;
Y = CH₂O oder CO-O-R¹-O;
R¹ = CH₂CH₂ oder CH₂-CHOH-CH₂;
P = CH₂=C(CH₃)-CO;
Z = H oder COOH;
R⁴ = CH₃, C₂H₅ oder Phenyl;
U = COOH;
R⁵ = CH₃, C₂H₅ oder Phenyl;
V = H oder COOH;
R² = CH₃ oder C₂H₅;
T = O;
R³ = CH₃ oder Phenyl;
a = 0,10 bis 0,80;
b = 0 bis 0,80; und/oder
c = 0 bis 0,60.

3. Verfahren zur Herstellung des Polymers gemäß Anspruch 1 oder 2, **dadurch gekennzeichnet, daß** man Mischungen von gegebenenfalls geschützten bicyclischen Verbindungen in Gegenwart eines Katalysators einer ringöffnenden Metathesepolymerisation unterzieht und anschließend eventuell vorhandene Schutzgruppen abspaltet, wobei die Mischungen
(a) die bicyclische Verbindung der allgemeinen Formel (II) und
(b) gegebenenfalls die bicyclische Verbindung der allgemeinen Formel (III)
und
(c) gegebenenfalls die bicyclische Verbindung der allgemeinen Formel (IV) 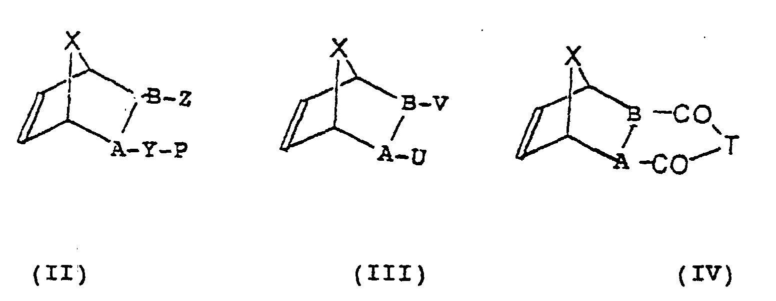 enthalten.

4. Verwendung des Polymers gemäß Anspruch 1 oder 2 als Bestandteil von Zementen, Beschichtungsmaterialien, Kompositen und Adhäsiven.

5. Verwendung des Polymers gemäß Anspruch 1 oder 2 als Dentalmaterial oder Bestandteil von Dentalmaterial, insbesondere als Bestandteil von Dentaladhäsiven.

6. Zusammensetzung mit Gehalt an dem polymerisierbaren Polymer gemäß Anspruch 1 oder 2.

7. Zusammensetzung nach Anspruch 6, **dadurch gekennzeichnet, daß** sie ein Zement, Beschichtungsmaterial, Komposit oder Adhäsiv ist.

8. Zusammensetzung nach Anspruch 6, **dadurch gekennzeichnet, daß** sie ein Dentalmaterial und insbesondere ein Dentinadhäsiv ist.

9. Zusammensetzung nach einem der Ansprüche 6 bis 8, **dadurch gekennzeichnet, daß** sie das polymerisierbare Polymer in zumindest teilweise polymerisierter Form enthält.

## Claims

1. Functionalised and polymerisable polymer **characterised in that** it contains the following repeat units (IA), (IB) and (IC): 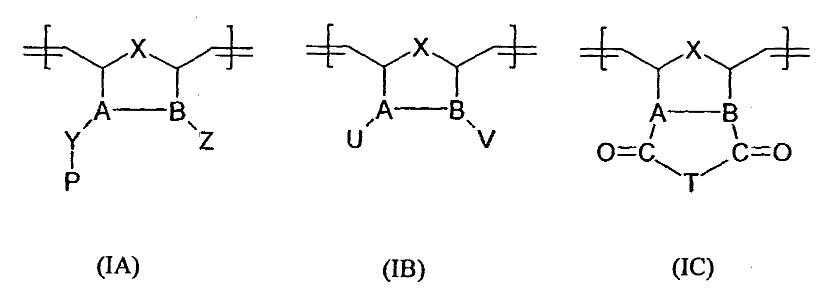 where X, A-B, Y, P, Z; U, V, T, R¹, R², R³, R⁴, and R⁵ independently of one another have the following meanings:
X = CH₂ or O;
A-B = C - C or C = C;
Y = CH₂O, CO-O or COO-R¹-O,
where R¹ is a substituted or unsubstituted C₁ to C₅ alkylene or oxyalkylene;
P = a polymerisable group, namely CH₂ = CH-CO-, CH₂ = C(CH₃)-CO-, CH₂ = CHCH₂-, or CH₂ = CH-C₆H₅-CH₂-;
Z = H, COOH, substituted or unsubstituted C₁ to C₁₂ alkyl or COOR⁴,
where R⁴ = a substituted or unsubstituted C₁ to C₁₂ alkyl or C₆ to C₁₄ aryl;
U = COOH or COOR⁵,
where R⁵ = a substituted or unsubstituted C₁ to C₁₂ alkyl or C₆ to C₁₄ aryl;
V = H, COOH, CH₂-OH, OR² or CO-OR²,
where R² = a substituted or unsubstituted C₁ to C₁₂ alkyl or C₆ to C₁₄ aryl;
T = O, NH, or NHR³,
where R³ = a substituted or unsubstituted C₁ to C₁₂ alkyl or C₆ to C₁₄ aryl;
and
where the mole fraction a of the unit (IA), the mole fraction b of the unit (IB) and the mole fraction c of the unit (IC) are as follows:
a = 0.05 to 1.0;
b = 0 to 0.95;
c = 0 to 0.90.

2. Polymer according to Claim 1 **characterised in that** the variables independently of one another have the following meaning:
X = CH₂ or O;
A-B = C-C;
Y = CH₂O or CO-O-R¹-O;
R¹ = CH₂CH₂ or CH₂-CHOH-CH₂;
P = CH₂ = C(CH₃)-CO;
Z = H or COOH;
R⁴ = CH₃, C₂H₅ or phenyl;
U = COOH;
R⁵ = CH₃, C₂H₅ or phenyl;
V = H or COOH;
R² = CH₃ or C₂H₅ ;
T = O;
R³ = CH₃ or phenyl;
a = 0.10 to 0.80;
b = 0 to 0.80; and/or
c = 0 to 0.60.

3. Process for the preparation of the polymer according to Claim 1 or 2, **characterised in that** mixtures of optionally protected bicyclic compounds are subjected to a ring -opening metathesis polymerisation in the presence of a catalyst, any protective groups are subsequently split off and wherein the mixtures comprise
(a) the bicyclic compound of the general formula (II) and
(b) optionally the bicyclic compound of the general formula (III)
and
(c) optionally the bicyclic compound of the general formula (IV).

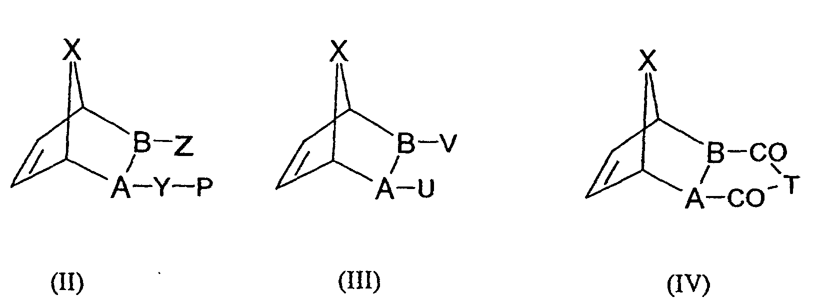

4. Use of the polymer according to Claim 1 or 2 as a constituent of cements, coating materials, composites and adhesives.

5. Use of the polymer according to Claim 1 or 2 as a dental material or a constituent of dental material, in particular as a constituent of dental adhesives.

6. Composition containing the polymerisable polymer according to Claim 1 or 2.

7. Composition according to Claim 6, **characterised in that** it is a cement, coating material, composite or adhesive.

8. Composition according to Claim 6, **characterised in that** it is a dental material and in particular a dentine adhesive.

9. Composition according to one of Claims 6 to 8, **characterised in that** it contains the polymerisable polymer in at least partially polymerised form.

## Revendications

1. Polymère fonctionnalisé et polymérisable, **caractérisé en ce qu'**il comporte les motifs répétitifs (IA), (IB) et (IC) suivants : 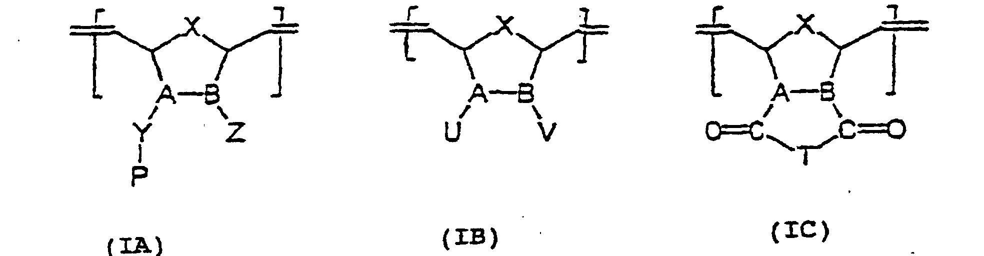 dans lesquels X, A-B, Y, P, Z, U, V, T, R¹, R², R³, R⁴ et R⁵ ont indépendamment les uns des autres les significations suivantes :
X = CH₂ ou O ;
A-B = C-C- ou C=C ;
Y = CH₂O, CO-O ou COO-R¹-O,
R¹ représentant un groupe alkylène ou oxyalkylène en C₁-C₅ substitué ou non substitué ;
P = un groupe polymérisable, à savoir CH₂=CH-CO-, CH₂=C(CH₃)-CO-, CH₂=CH-CH₂- ou CH₂=CH-C₆H₅-CH₂- ;
Z = H, COOH, un groupe alkyle en C₁-C₁₂ substitué ou non substitué ou COOR⁴,
R⁴ représentant un groupe alkyle en C₁-C₁₂ ou aryle en C₆-C₁₄ substitué ou non substitué ;
U = COOH ou COOR⁵,
R⁵ représentant un groupe alkyle en C₁-C₁₂ ou aryle en C₆-C₁₄ substitué ou non substitué ;
V = H, COOH, CH₂-OH, OR² ou CO-OR²,
R² représentant un groupe alkyle en C₁-C₁₂ ou aryle en C₆-C₁₄ substitué ou non substitué ; et
T = O, NH ou NHR³,
R³ représentant un groupe alkyle en C₁-C₁₂ ou aryle en C₆-C₁₄ substitué ou non substitué ;
et
la fraction molaire a du motif (IA), la fraction molaire b du motif (IB) et la fraction molaire c du motif (IC) étant comme suit :
a = 0,05 à 1,0 ;
b = 0 à 0,95 et
c = 0 à 0,90.

2. Polymère selon la revendication 1, **caractérisé en ce que** les variables ont indépendamment les unes des autres les significations suivantes :
X = CH₂ ou O ;
A-B = C-C ;
Y = CH₂O ou CO-O-R¹-O ;
R¹ = CH₂CH₂ ou CH₂-CHOH-CH₂ ;
P = CH₂=C(CH₃)-CO ;
Z = H ou COOH ;
R⁴ = CH₃, C₂H₅ ou phényle ;
U = COOH ;
R⁵ = CH₃, C₂H₅ ou phényle ;
V = H ou COOH ;
R² = CH₃ ou C₂H₅ ;
T = O ;
R³ = CH₃ ou phényle ;
a = 0,10 à 0,80 ;
b = 0 à 0,80 et/ou
c = 0 à 0,60.

3. Procédé pour la préparation du polymère selon la revendication 1 ou 2, **caractérisé en ce qu'**on soumet des mélanges de composés bicycliques éventuellement protégés à une polymérisation par métathèse avec ouverture de cycle, en présence d'un catalyseur, et que l'on élimine ensuite les groupes protecteurs éventuellement présents, les mélanges contenant
(a) le composé bicyclique de formule générale (II) et
(b) éventuellement le composé bicyclique de formule générale (III) et
(c) éventuellement le composé bicydique de formule générale (IV)

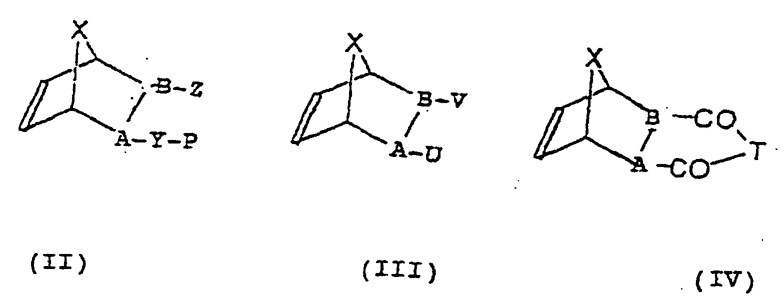

4. Utilisation du polymère selon la revendication 1 ou 2, comme composant de ciments, matériaux de revêtement, composites et adhésifs.

5. Utilisation du polymère selon la revendication 1 ou 2, comme matériau dentaire ou composant de matériau dentaire, en particulier comme composant d'adhésifs dentaires.

6. Composition ayant une teneur en polymère polymérisable selon la revendication 1 ou 2.

7. Composition selon la revendication 6, **caractérisée en ce qu'**elle est un ciment, un matériau de revêtement, un composite ou un adhésif.

8. Composition selon la revendication 6, **caractérisée en ce qu'**elle est un matériau dentaire et en particulier un adhésif dentaire.

9. Composition selon l'une quelconque des revendications 6 à 8, **caractérisée en ce qu'**elle contient le polymère polymérisable sous forme au moins partiellement polymérisée.
